# EUROPEAN PATENT SPECIFICATION

(11) **EP 1 438 277 B1**
(45) Date of publication and mention of the grant of the patent: **07.03.2007**
(21) Application number: 01954786.8
(22) Date of filing: 19.07.2001
(51) Int. Cl.: C07C 7/20, C07C 15/46, C07B 63/04, C09K 15/30

(54) **RECYCLE OF NITROXYL-CONTAINING STREAMS AT LOW TEMPERATURE**
WIEDERGEWINNUNG VON NITROXYL-ENTHALTENDEN STRÖMEN BEI NIEDRIGER TEMPERATUR
RECYCLAGE DE COURANTS CONTENANT DU NITROXYLE A BASSE TEMPERATURE

(30) Priority: 02.08.2000 US 222595 P
(43) Date of publication of application: 21.07.2004
(73) Proprietor: UNIROYAL CHEMICAL COMPANY, Inc., Middlebury, CT 06749 (US)
(72) Inventor: BENAGE, Brigitte, Wolcott, CT 06715 (US); ABRUSCATO, Gerald, J., Southington, CT 06489 (US); EISENSTEIN, Andrew, J., Southbury, CT 06488 (US); SCHLUP, Kirk, A., Woodbury, CT 06798 (US); GREWAL, Ruben, S., Oakville, CT 06779 (US); GEELAN, Brendan, East Haven, CT 06613 (US)
(74) Representative: Spott, Gottfried
(86) International application number: PCT/US2001/022771
(87) International publication number: WO 2002/010100

(56) References cited:
- EP-A- 0 178 168
- EP-A- 0 959 059
- US-A- 5 254 760
- CHEMICAL ABSTRACTS, vol. 132, no. 13, 27 March 2000 (2000-03-27) Columbus, Ohio, US; abstract no. 166750, KASHIHARA, KUNIO ET AL: "Improvement of monomer recovery in preparation of vinyl polymers" XP002188924 & JP 2000 053594 A (HAKUTO K. K., JAPAN) 22 February 2000 (2000-02-22)

## Description

### BACKGROUND OF THE INVENTION

### 1. Field of the Invention

The present invention relates to the inhibition of polymerization and polymer growth of ethylenically unsaturated monomers by means of the addition thereto of at least one stable nitroxide free radical compound.

### 2. Description of Related Art

Many ethylenically unsaturated monomers undesirably polymerize at various stages of their manufacture, processing, handling, storage, and use. Polymerization, such as thermal polymerization, during their purification results in the loss of the monomer, i.e., a lower yield, and an increase in the viscosity of any tars that may be produced. The processing and handling of the higher viscosity tars then requires higher temperature and work (energy cost) to remove residual monomer.

Polymerization can also result in equipment fouling, especially in the case of production of acrylic monomers. Such polymerization causes loss in production efficiency owing to the deposition of polymer in or on the equipment being used. These deposits must be removed from time to time, leading to additional loss in production of the monomer.

A wide variety of compounds has been proposed and used for inhibiting uncontrolled and undesired polymerization of ethylenically unsaturated monomers. However, many of these compounds have not been fully satisfactory.

There are several mechanisms by which polymerization inhibitors work. One mode of action for polymerization inhibitors is for the inhibiting species to combine with the propagating polymer chain such that the polymerization of that polymer chain stops, i.e., a termination reaction. If such an inhibitor-terminated polymer chain is capable of participating in a dynamic equilibrium between a dormant species (the inhibitor-terminated chain) and an active polymer chain, it would be considered a "living" or quasiliving polymer. For example, Ivan, *Macromol. Symp. 88*:201-215 (1994) describes quasiliving polymerization as a polymerization in which "... only a portion of chain ends are active (propagating) and these are in equilibria with inactive (dormant, nonpropagating) chains..." Shigemoto *et al., Macromol. Rapid Commun. 17*:347-351 (1996) state, "Well-defined polymers can be prepared by controlled / "living" radical polymerization in the presence of relatively stable radicals. These systems employ the principle of dynamic equilibration between dormant species and growing radicals via reversible homolytic cleavage of a covalent bond in dormant species." Further, Greszta *et al., Macromolecules 29*:7661-7670 (1996) state, "The reversible homolytic cleavage of dormant species can be accomplished by either thermal, photochemical, or catalytic activation. The most successful approaches are as follows: homolytic cleavage of alkoxyamines and dithiocarbamates, use of various organometallic species, and catalyzed atom transfer radical polymerization." Such a "living" polymer is capable of increasing in molecular weight (growing) through its reaction with additional monomer units of the same or different types of polymerizable monomers.

The method by which this "living" polymer grows is termed the "living" polymerization mechanism, and is depicted below.

M-Inh ----- > M* + *Inh (1)

M* + *Inh ----> M-Inh (2)

M* +M' ----> M-M'* (3)

M-M'* + *Inh ----> M-M'-Inh (4)

Reactions (1) and (2) depict the dynamic equilibrium, with (2) being the termination reaction. Reaction (3) depicts growth of the polymer chain. Reaction (4) depicts re-termination of the growing polymer chain with the inhibiting species. The amount of growth over any period of time is dependent on the relative rate at which (2) occurs versus (3), as long as (1) occurs to some extent. The faster (2) is relative to (3), the more time is needed for significant growth of the polymer. Under the conditions in which inhibitors are normally used, the concentration of the inhibiting species should be sufficiently high to cause reaction (2) to be much faster than reaction (3), otherwise it would not be an effective inhibiting system for commercial use. However, we have realized that even at an effective inhibiting amount of the inhibitor, growth can still occur, given sufficient time and temperature.

There are at least two scenarios in which "living" polymer can remain in a monomer purification train for an excessive amount of time.

First, the use of tar recycle can significantly increase the amount of time that the "living" polymer can remain in the purification train. To recycle unused inhibitor that is left in the purification stream after removal of the monomer, a portion of the residual (or tar) stream is added to a feed stream earlier in the purification train. This residual stream typically contains inhibitor, small amounts of monomer, impurities in the monomer stream that have been concentrated by the purification process, and polymer formed during the production and purification process. Recycling this polymer will allow it time to grow if it is "living" polymer and the conditions of the purification train allow the "living" polymerization mechanism to occur. If this polymer grows via the "living" polymerization mechanism, excessive polymerization would cause loss in product yield, increased waste residues from the process, and potential plugging of equipment due to excessively high molecular weight polymer in the purification stream.

Second, occasionally, conditions in the plant/purification process can result in the formation of polymer within the purification train that is not dissolved by the monomer stream. If this polymer is caught in a dead space, or if it attaches to the metal on the inside of the equipment, it will not be washed out of the system. Thus, the polymer will remain within the system indefinitely (potentially for two or more years). If this polymer grows via the "living" polymerization mechanism, it could coat the inside of the equipment, causing inefficient separation of the monomer stream components and/or insufficient or inefficient heat transfer to enable purification. Such a situation would cause loss in product yield and could potentially cause an unscheduled shut-down of the plant in order to clean out the undissolved polymer in the equipment. Such a shut-down results in loss of monomer production and additional expense to clean out and dispose of the undissolved polymer.

It is significant that there has been no indication that previously used inhibitors would lead to the formation of "living" polymer when used as polymerization inhibitors. However, a newly utilized class of inhibitors, the stable nitroxyl radicals, is known to allow this "living" polymerization mechanism to occur. These nitroxyl radicals are highly efficient polymerization inhibitors under normal use, providing better performance than most other inhibitors on the market, but their incapacity to prevent "living" polymerization has hindered their full utilization. Accordingly, there is a need for improved techniques that can be employed in a purification train whereby nitroxyl radicals can be used to full advantage to prevent the polymer growth that occurs via a "living" polymerization mechanism.

Nitroxyl radicals are known to facilitate polymerization via a "living" free radical process to give polymers of narrow polydispersity.

Georges *et al., Macromolecules 26(11)*:2987-2988 (1993) synthesized narrow molecular weight resins by a free-radical polymerization process with polydispersities comparable to those that can be obtained by anionic polymerization processes and below the theoretical limiting polydispersity of 1.5 for a conventional free-radical polymerization process. The process comprised heating a mixture of monomer(s), free-radical initiator, and a stable free radical, e.g., 2,2,6,6-tetramethyl-1-piperidinyloxy (TEMPO).

Hawker *et al., Macromolecules 29(16)*:5245-5254 (1996) prepared and evaluated a variety of initiating systems for the preparation of macromolecules by nitroxide-mediated "living" free radical procedures. The systems were divided into two classes, unimolecular initiators in which alkylated TEMPO derivatives dissociate to provide both the initiating radical and the stable radical, and bimolecular systems in which a traditional free radical initiator, such as BPO or AIBN, is used in conjunction with TEMPO. For the unimolecular initiators it was found that an α-methyl group is essential for "living" character, while a variety of substituents could be placed on the phenyl ring or the β-carbon atom without affecting the efficiency of the unimolecular initiator. It was found that the rate of polymerization is approximately the same for both the unimolecular and corresponding bimolecular systems; however, the unimolecular initiators afforded better control over molecular weight and polydispersity.

Hindered nitroxyl compounds are known to be very active inhibitors of free radical polymerizations of unsaturated monomers such as styrene, acrylic acid, methacrylic acid, and the like. Nitrophenols, nitrosophenols, phenylenediamines (PDA's), hydroxylamines, quinones and hydroquinones are also known to have a similar capacity.

U.S. Patent Number 3,163,677 discloses a process for the preparation of N,N,O-trisubstituted hydroxylamines and N,N-disubstituted nitroxides of the formulae: 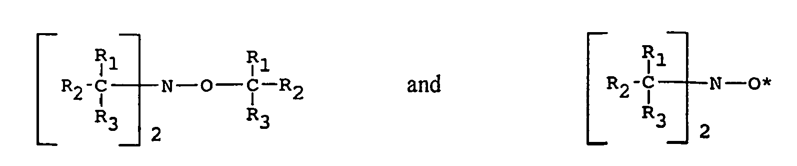 wherein R₁, R₂, and R₃ are each an alkyl radical having 1 to 15 carbon atoms. (As used herein, the designation N-O* denotes a stable free radical wherein the asterisk is an unpaired electron.) The N,N,O-trisubstituted hydroxylamines can be used to make the N,N-disubstituted nitroxides, which are stable free radicals and are said to be useful as polymerization inhibitors.

U.S. Patent Number 3,334,103 discloses that nitroxides can be prepared from the corresponding heterocyclic amine wherein the nitrogen atom of the nitroxide group is attached to other than a tertiary carbon of an aliphatic group (i.e., the nitrogen atom forms a part of a heterocyclic nucleus). These nitroxides are said to have useful properties similar to those described for the N,N-disubstituted nitroxides ofU. S. Patent Number 3,163,677.

U.S. Patent Number 3,372,182 discloses that a great variety ofN,N-disubstituted, stable, free radical nitroxides not otherwise readily available can be prepared by a simple and convenient process that comprises pyrolyzing in an inert reaction medium virtually any hydroxylamine that is susceptible to cleavage of the O-C bond, e.g., tri-t-butylhydroxylamine.

U.S. Patent Number 3,422,144 discloses stable, free radical nitroxides of the formula: 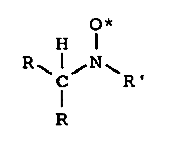 wherein R is selected from the group consisting of tertiary alkyl, aryl, alkaryl, haloaryl, carboxyaryl, alkoxyaryl, alkylthioaryl, pyridyl, and dialkylaminoaryl, and R' is tertiary alkyl. These nitroxides are said to be useful as traps for reactive free radicals both in the counting of free radicals and for inhibiting oxidation and free radical polymerization.

U.S. Patent Number 3,494,930 discloses free radicals of the nitroxide type for use as initiators of free radical reactions, collectors of free radicals, polymerization inhibitors or antioxidants. They are constituted by nitrogenous bicyclic compounds in which one of the bridges comprises solely the nitroxide radical group and, in particular, by aza-9-bicyclo (3,3,1) nonanone-3-oxyl-9, and by aza-9-bicyclo (3,3,1) nonane oxyl-9.

U.S. Patent Number 3,873,564 discloses compounds and a method for assaying enzymes by adding to a medium containing an enzyme a stable free radical compound having a stable free radical functionality which, when subjected to an enzyme-catalyzed reaction, changes the environment of the free radical functionality. By following the change in the electron spin resonance spectrum as affected by the change in environment, the type of enzyme and the activity of the enzyme can be determined. The compounds found useful are normally stable nitroxide radicals with an enzyme labile functionality. Other compounds include two cyclic nitroxide containing rings joined by a chain having an enzyme labile functionality.

U.S. Patent Number 3,959,395 discloses a process for recovery and re-use of dinitrophenols employed as inhibitors of polymerization in the distillation and purification of styrene. The styrene still residues commonly referred to as tar or tars are treated with an aqueous hydroxide at a controlled pH, the phases are separated, the aqueous phase is treated with an acid and an organic solvent at a controlled pH and the resulting organic phase is recycled to a suitable point in the styrene purification system. The recycled solution is said to be a more effective inhibitor than the dinitrophenol originally employed.

U.S. Patent Number 3,966,711 teaches that 2,2,7,7-tetraalkyl- and 2,7-dispiroalkylene-5-oxo-1,4-diazacycloheptanes substituted in the 4-position by mono- or tetravalent radicals are powerful light-stabilizers for organic polymers. They are said to possess higher compatibility than their 4-unsubstituted homologues, from which they can be synthesized by reactions known for N-alkylation. Preferred substituents in the 4-position are alkyl, alkylene, alkenyl, aralkyl, and esteralkyl groups. The 1-nitroxyls derived from the imidazolidines by oxidation with hydrogen peroxide or percarboxylic acids are also said to be good light stabilizers.

U.S. Patent Number 4,033,829 discloses the inhibition of styrene against polymerization during the distillation thereof by incorporating therein, in an amount sufficient to inhibit polymerization thereof, the dinitrophenol solution recovered from styrene still residues or tars resulting from the distillation of styrene in the presence of dinitrophenol. The recovered dinitrophenol solution is said to be a more effective polymerization inhibitor than dinitrophenol per se.

U.S. Patent Numbers 4,252,615 and 4,469,558 disclose a process for the distillation of readily polymerizable vinyl aromatic compounds and a polymerization inhibitor therefor. The process comprises subjecting a vinyl aromatic compound to elevated temperatures in a distillation system in the presence of a polymerization inhibitor comprising 2,6-dinitro-p-cresol. Also disclosed is a distillation method and apparatus for use with this inhibitor.

U.S. Patent Number 4,665,185 discloses a process for the efficient preparation of nitroxyls of sterically hindered amines by the oxidation of the amine using a hydroperoxide in the presence of a small amount of a metal ion catalyst, at moderate temperature for a short period of time, to give the nitroxyl in high yield and purity.

U.S. Patent Number 4,774,374 discloses a vinyl aromatic composition stabilized against polymerization comprising (a) a vinyl aromatic compound and (b) an effective amount of a stabilizer system in which the active ingredient consists essentially of an oxygenated species formed by the reaction of oxygen and an N-aryl-N'-alkyl-p-phenylenediamine.

U.S. Patent Number 5,254,760 teaches that the polymerization of a vinyl aromatic compound, such as styrene, is very effectively inhibited during distillation or purification by the presence of at least one stable nitroxyl compound together with at least one aromatic nitro compound.

U.S. Patent Numbers 5,545,782 and 5,545,786 disclose that nitroxyl inhibitors in combination with some oxygen reduce the premature polymerization of vinyl aromatic monomers during the manufacturing processes for such monomers. Even small quantities of air used in combination with the nitroxyl inhibitors are said to result in vastly prolonged inhibition times for the monomers.

European Patent Application 0 178 168 A2 discloses a method for inhibiting the polymerization of an α,β-ethylenically unsaturated monocarboxylic acid during its recovery by distillation by using a nitroxide free radical.

European Patent Application 0 765 856 A1 discloses a stabilized acrylic acid composition in which the polymerization of the acrylic acid is inhibited during the distillation process for purifying or separating the acrylic acid as well as during transport and storage. The compositions comprise three components: (a) acrylic acid, (b) a stable nitroxyl radical, and (c) a dihetero-substituted benzene compound having at least one transferable hydrogen (e.g., a quinone derivative such as the monomethyl ether of hydroquinone (MEHQ)). During the distillation process, transport, and storage, components (b) and (c) are present in a polymerization-inhibiting amount. During the distillation process, oxygen (d) is preferably added with components (b) and (c). According to the specification, examples of suitable nitroxide free radical compounds include di-t-butylnitroxide; di-t-amylnitroxide; 2,2,6,6-tetramethyl-piperidinyloxy; 4-hydroxy-2,2,6,6-tetramethyl-piperidinyloxy; 4-oxo-2,2,6,6-tetramethyl-piperidinyloxy; 4-dimethylamino-2,2,6,6-tetramethyl-piperidinyloxy; 4-amino-2,2,6,6-tetramethyl-piperidinyloxy; 4-ethanoyloxy-2,2,6,6-tetramethyl-piperidinyloxy; 2,2,5,5-tetramethylpyrrolidinyloxy; 3-amino-2,2,5,5-tetramethylpyrrolidinyloxy; 2,2,5,5-tetramethyl-1-oxa-3-azacyclopentyl-3-oxy; 2,2,5,5-tetramethyl-1-oxa-3-pyrrolinyl-1-oxy-3-carboxylic acid; and 2,2,3,3,5,5,6,6-octamethyl-1,4-diazacyclohexyl-1,4-dioxy.

WO 97/46504 concerns substance mixtures containing: (A) monomers containing vinyl groups; and (B) an active amount of a mixture which inhibits premature polymerization of the monomers containing vinyl groups during their purification or distillation and contains: (i) between 0.05 and 4.5 wt %, relative to the total mixture (B), of at least one N-oxyl compound of a secondary amine which has no hydrogen atom at the α-C atoms; and (ii) between 99.95 and 95.5 wt % relative to the total mixture (B), of at least one nitro compound. The publication also discloses a process for inhibiting the premature polymerization of monomers, and the use of mixture (B) for inhibiting the premature polymerization of monomers.

WO 98/14416 discloses that the polymerization of vinyl aromatic monomers such as styrene is inhibited by the addition of a composition of a stable hindered nitroxyl radical and an oxime compound.

WO 98/25872 concerns substance mixtures containing: (A) compounds containing vinyl groups; (B) an active amount of a mixture which inhibits premature polymerization of the compounds containing vinyl groups and contains: (i) at least one N-oxyl compound of a secondary amine which does not carry any hydrogen atoms on the α-carbon atoms; and (ii) at least one iron compound; (C) optionally nitro compounds; and (D) optionally co-stabilizers. The publication also discloses a process for inhibiting the premature polymerization of compounds (A) containing vinyl groups, and the use of (B) optionally mixed with nitro compounds (C) and/or co-stabilizers (D) for inhibiting the premature polymerization of radically polymerizable compounds and stabilizing organic materials against the harmful effect of radicals.

U.K. Patent Number 1,127,127 discloses that acrylic acid can be stabilized against polymerization by the addition thereto of a nitroxide having the essential skeletal structure: 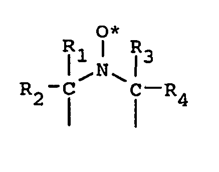 wherein R₁, R₂, R₃, and R₄ are alkyl groups and no hydrogen is bound to the remaining valencies on the carbon atoms bound to the nitrogen. The two remaining valencies that are not satisfied by R₁ to R₄ or nitrogen can also form part of a ring (e.g., 2,2,6,6 tetramethyl-4-hydroxy-piperidine-1-oxyl).

UK Patent Application GB 2 069 523 A discloses carrying out the distillation of readily polymerizable vinyl aromatic compounds at elevated temperature in the presence of a polymerization inhibitor comprising 2,6-dinitro-*p*-cresol.

CS-260755 B1 is directed to the preparation of 4-substituted-2,2,6,6-tetramethylpiperidine nitroxyls as olefin stabilizers.

SU-334845 A1 is directed to the inhibition of the radical polymerization of oligoester acrylates using iminoxyl radical inhibitors of a given formula.

FR 2,761,060 relates to the prevention of premature polymerization of styrene during its production by dehydrogenation of ethylbenzene by injecting into the process effluent a radical inhibitor based on an oxyl-tetramethylpiperidine derivative.

The foregoing are incorporated herein by reference in their entirety.

### SUMMARY OF THE INVENTION

During the production and purification of unsaturated monomers, many manufacturers recycle certain process streams containing the polymerization inhibitor in order to improve the economics of the process by reusing the inhibitor left in the process stream. It is known in the industry that the recycling of streams utilizing nitroxyls as polymerization inhibitors in plants employing temperatures in excess of about 115° C causes loss of inhibitor efficiency, such that the tar recycle leads to a higher polymer content than would be expected or desirable.

It has now been discovered that recycling streams utilizing nitroxyls as polymerization inhibitors can be done effectively in some plants without loss of inhibitor efficiency.

More particularly, the present invention is directed to an improvement in a process for the production and purification of unsaturated monomers employing nitroxyl-containing inhibitors wherein process streams containing the inhibitor are recycled, wherein said streams are recycled at a reboiler temperature below 110° C.

### DESCRIPTION OF THE PREFERRED EMBODIMENTS

As stated above, the present invention is directed to inhibiting systems in which at least one nitroxyl radical is used in the purification train to prevent polymer growth that occurs via a "living" polymerization mechanism and, more particularly, to effectively recycling the nitroxyl-containing process streams at reboiler temperatures below 110° C without loss of inhibitor efficiency.

In one preferred aspect, the inhibitor is a stable hindered nitroxyl compound having the structural formula: 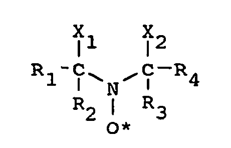 wherein
R₁ and R₄ are independently selected from the group consisting of hydrogen, alkyl, and heteroatom-substituted alkyl;
R₂ and R₃ are independently selected from the group consisting of alkyl and heteroatom-substituted alkyl; and
X₁ and X₂
(1) are independently selected from the group consisting of halogen, cyano, amido, -S-C₆H₅, carbonyl, alkenyl, alkyl of 1 to 15 carbon atoms, COOR₇, -S-COR₇, and -OCOR₇, wherein R₇ is alkyl or aryl, or
(2) taken together, form a ring structure with the nitrogen.

In a particularly preferred embodiment, the stable hindered nitroxyl compound has the structural formula: 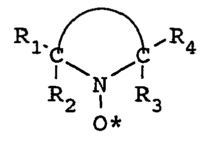 wherein R₁ and R₄ are independently selected from the group consisting of hydrogen, alkyl, and heteroatom-substituted alkyl and R₂ and R₃ are independently selected from the group consisting of alkyl and heteroatom-substituted alkyl, and the 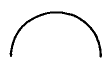 portion represents the atoms necessary to form a five-, six-, or seven-membered heterocyclic ring.

Accordingly, one of the several classes of cyclic nitroxides that can be employed in the practice of the present invention can be represented by the following structural formula: 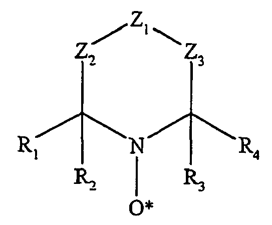 wherein Z₁, Z₂, and Z₃ are independently selected from the group consisting of oxygen, sulfur, secondary amines, tertiary amines, phosphorus of various oxidation states, and substituted or unsubstituted carbon atoms, such as >CH₂, >CHCH₃, >C=O, >C(CH₃)₂, >CHBr, >CHCl, >CHI, >CHF, >CHOH, >CHCN, >C(OH)CN, >CHCOOH, >CHCOOCH₃, >CHCOOC₂H₅, >C(OH)COOC₂H₅, >C(OH)COOCH₃, >C(OH)CHOHC₂H₅, >CR₅OR₆, >CHNR₅R₆, >CCONR₅R₆, >C=NOH, >C=CH-C₆H₅, >CF₂, >CCl₂, >CBr₂, >CI₂, >CR₅PR₁₃R₁₄R₁₅, and the like, where R₅ and R₆ are independently selected from the group consisting of hydrogen, alkyl, aryl, and acyl and R₁₃, R₁₄, and R₁₅ are independently selected from the group consisting of unshared electrons, alkyl, aryl, =O, OR₁₆, and NR₁₇R₁₈, where R₁₆, R_{17,} and R₁₈ are independently selected from the group consisting of hydrogen, alkyl, and aryl. Where R₅ and/or R₆ are alkyl, it is preferred that they be a lower alkyl (i.e., one having one to five carbon atoms, e.g., methyl, ethyl, propyl, butyl, pentyl, and isomers thereof).

Where R₅ and/or R₆ are aryl, it is preferred that they be aryl of from 6 to 10 carbon atoms, e.g., phenyl or naphthyl, which, in addition, may be substituted with non-interfering substituents, e.g., lower alkyl groups, halogens, and the like.

Where R₅ and/or R₆ are acyl, it is preferred that they be acyl of the structure 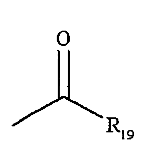 where R₁₉ is alkyl, aryl, OR₂₀, or NR₂₀R₂₁ and where R₂₀ and R₂₁ are alkyl, aryl, or 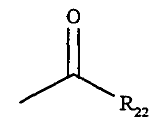 where R₂₂ is alkyl or aryl. Where R₁₉, R₂₀, R₂₁, or R₂₂ are alkyl, they are preferably alkyl of from 1 to 15 carbon atoms, more preferably lower alkyl of from 1 to 5 carbon atoms, as described above. Where R₁₉, R₂₀, R₂₁, or R₂₂ are aryl, they are preferably aryl of from 6 to 10 carbon atoms, as described above.

Another of the several classes of cyclic nitroxides that can be employed in the practice of the present invention can be represented by the following structural formula: 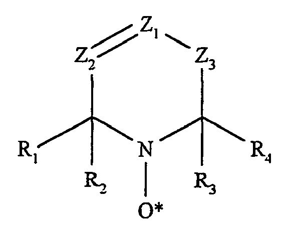 wherein Z₁ and Z₂, which may be the same or different, are nitrogen or substituted or unsubstituted carbon atoms, such as =C(H)-, =C(CH₃)-, =C(COOH)-, =C(COOCH₃)-, =C(COOC₂H₅)-, =C(OH)-, =C(CN)-, =C(NR₅R₆)-, =C(CONR₅R₆)-, and the like, and where Z₃, R₅, and R₆ are as described above.

The cyclic nitroxides employed in the practice of the present invention can also be derived from five-membered rings. These compounds are of the structure: 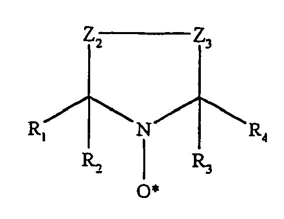 wherein Z₂ and Z₃, which may be the same or different, are sulfur, oxygen, secondary amines, tertiary amines, phosphorus of various oxidation states, or substituted or unsubstituted carbon atoms, such as, >CH₂, >CHCH₃, >C=O, >C(CH₃)₂, >CHBr, >CHCl, >CHI, >CHF, >CHOH, >CHCN, >C(OH)CN, >CHCOOH, >CHCOOCH₃, >CHCOOC₂H₅, >C(OH)COOC₂H₅, >C(OH)COOCH₃, >C(OH)CHOHC₂H₅, >CR₅OR₆, >CHNR₅R₆, >CCONR₅R₆, >C=NOH, >C=CH-C₆H₅, CF₂, CCl₂, CBr₂, CI₂, >CR₅PR₁₃R₁₄R₁₅, and the like, wherein the several R groups are as described above.

The cyclic nitroxides employed in the practice of the present invention can also have the structure: 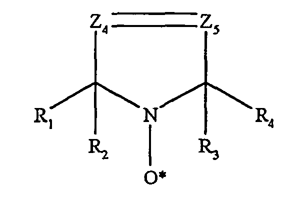 wherein Z₄ and Z₅, which can be the same or different, can be nitrogen or a substituted or unsubstituted carbon atom, such as =C(H)-, =C(CH₃)-, =C(COOH)-, =C(COOCH₃)-, =C(COOC₂H₅)-, =C(OH)-, =C(CN)-, =C(NR₅R₆)-, =C(CONR₅R₆)-,
and the like, where R₅ and R₆ are as described above.

Another class of cyclic nitroxides that can be employed in the practice of the present invention is of the structure: 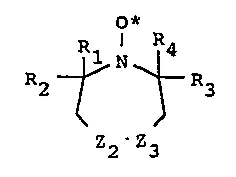 wherein Z₂ and Z₃, which may be the same or different, are sulfur, oxygen, secondary amines, tertiary amines, or substituted or unsubstituted carbon atoms, such as, >CH₂, >CHCH₃, >C=O, >C(CH₃)₂, >CHBr, >CHCl, >CHI, >CHF, >CHOH, >CHCN, >C(OH)CN, >CHCOOH, >CHCOOCH₃, >CHCOOC₂H₅, >C(OH)COOC₂H₅, >C(OH)COOCH₃, >C(OH)CHOHC₂H₅, >CHNR₅R₆,>CCONR₅R₆, >CR₅OR₆, >C=NOH, >C=CH-C₆H₅, CF₂, CCl₂, CBr₂, CI₂, >CR₅PR₁₃R₁₄R₁₅, and the like, where the several R groups are as described above.

Further, two or more nitroxyl groups can be present in the same molecule, for example, by being linked through one or more of the Z-type moieties by a linking group E, as disclosed in U.S. Patent Number 5,254,760, which is incorporated herein by reference.

As stated above, for all the nitroxyl structures above, R₁ and R₄ are independently selected from the group consisting of hydrogen, alkyl, and heteroatom-substituted alkyl and R₂ and R₃ are independently selected from the group consisting of alkyl and heteroatom-substituted alkyl. The alkyl (or heteroatom-substituted alkyl) groups R₁ through R₄ can be the same or different and preferably contain 1 to 15 carbon atoms, e.g., methyl, ethyl, propyl, butyl, pentyl, hexyl, heptyl, octyl, nonyl, decyl, undecyl, dodecyl, tridecyl, tetradecyl, pentadecyl, and the like, and isomers thereof, e.g., t-butyl, 2-ethylhexyl, and the like. It is more preferred that R₁ through R₄ be independently selected from lower alkyls (or heteroatom-substituted lower alkyls) of one to five carbon atoms (e.g., methyl, ethyl, propyl, butyl, pentyl, and isomers thereof). Where heteroatom substituents are present, they can, for example, include halogen, oxygen, sulfur, nitrogen, and the like. It is most preferred that all of R₁ through R₄ be methyl.

Examples of suitable nitroxide free radical compounds that can be used in the practice of the present invention, include, but are not limited to:
N,N-di-*tert*-butylnitroxide;
N,N-di-*tert*-amylnitroxide;
N-*tert*-butyl-2-methyl-1-phenyl-propylnitroxide;
N-*tert*-butyl-1-diethylphosphono-2,2-dimethylpropylnitroxide;
2,2,6,6-tetramethyl-piperidinyloxy;
4-amino-2,2,6,6-tetramethyl-piperidinyloxy;
4-hydroxy-2,2,6,6-tetramethyl-piperidinyloxy;
4-oxo-2,2,6,6-tetramethyl-piperidinyloxy;
4-dimethylamino-2,2,6,6-tetramethyl-piperidinyloxy;
4-ethanoyloxy-2,2,6,6-tetramethyl-piperidinyloxy;
2,2,5,5-tetramethylpyrrolidinyloxy;
3-amino-2,2,5,5-tetramethylpyrrolidinyloxy;
2,2,4,4-tetramethyl-1-oxa-3-azacyclopentyl-3-oxy;
2,2,4,4-tetramethyl-1-oxa-3-pyrrolinyl-1-oxy-3-carboxylic acid;
2,2,3,3,5,5,6,6-octamethyl-1,4-diazacyclohexyl-1,4-dioxy;
4-bromo-2,2,6,6-tetramethyl-piperidinyloxy;
4-chloro-2,2,6,6-tetramethyl-piperidinyloxy;
4-iodo-2,2,6,6-tetramethyl-piperidinyloay;
4-fluoro-2,2,6,6-tetramethyl-piperidinyloxy;
4-cyano-2,2,6,6-tetramethyl-piperidinyloxy;
4-carboxy-2,2,6,6-tetramethyl-piperidinyloxy;
4-carbomethoay-2,2,6,6-tetramethyl-piperidinyloxy;
4-carbethoxy-2,2,6,6-tetramethyl-piperidinyloxy;
4-cyano-4-hydroxy-2,2,6,6-tetramethyl-piperidinyloxy;
4-methyl-2,2,6,6-tetramethyl-piperidinyloxy;
4-carbethoxy-4-hydroxy-2,2,6,6-tetramethyl-piperidinyloxy;
4-hydroxy-4-(1-hydroxypropyl)-2,2,6,6-tetramethyl-piperidinyloxy;
4-methyl-2,2,6,6-tetramethyl-1,2,5,6-tetrahydropyridine -1-oxyl;
4-carboxy-2,2,6,6-tetramethyl-1,2,5,6-tetrahydropyridine -1-oxyl;
4-carbomethoxy-2,2,6,6-tetramethyl-1,2,5,6-tetrahydropyridine -1-oxyl;
4-carbethoxy-2,2,6,6-tetramethyl-1,2,5,6-tetrahydropyridine -1-oxyl;
4-amino-2,2,6,6-tetramethyl-1,2,5,6-tetrahydropyridine-1-oxyl;
4-amido-2,2,6,6-tetramethyl-1,2,5,6-tetrahydropyridine-1-oxyl;
3,4-diketo-2,2,5,5-tetramethylpyrrolidinyloxy;
3-keto-4-oximino-2,2,5,5-tetramethylpyrrolidinyloxy;
3-keto-4-benzylidine-2,2,5,5-tetramethylpyrrolidinyloxy;
3-keto-4,4-dibromo-2,2,5,5-tetramethylpyrrolidinyloxy;
2,2,3,3,5,5-hexamethylpyrrolidinyloxy;
3-carboximido-2,2,5,5-tetramethylpyrrolidinyloxy;
3-oximino-2,2,5,5-tetramethylpyrrolidinyloxy;
3 -hydroxy-2,2,5,5-tetramethylpyrrolidinyloxy;
3-cyano-3-hydroxy-2,2,5,5-tetramethylpyrrolidinyloxy;
3-carbomethoxy-3-hydroxy-2,2,5,5-tetramethylpyrrolidinyloxy;
3-carbethoxy-3-hydroxy-2,2,5,5-tetramethylpyrrolidinyloxy;
2,2,5,5-tetramethyl-3-carboxamido-2,5-dihydropyrrole-1-oxyl;
2,2,5,5-tetramethyl-3-amino-2,5-dihydropyrrole-1-oxyl;
2,2,5,5-tetramethyl-3-carbethoxy-2,5-dihydropyrrole-1-oxyl;
2,2,5,5-tetramethyl-3-cyano-2,5-dihydropyrrole-1-oxyl;
bis(1-oxyl-2,2,6,6-tetramethylpiperidin-4-yl)succinate;
bis(1-oxyl-2,2,6,6-tetramethylpiperidin-4-yl)adipate;
bis(1-oxyl-2,2,6,6-tetramethylpiperidin-4-yl)sebacate;
bis(1-oxyl-2,2,6,6-tetramethylpiperidin-4-yl)n-butylmalonate;
bis(1-oxyl-2,2,6,6-tetramethylpiperidin-4-yl)phthalate;
bis(1-oxyl-2,2,6,6-tetramethylpiperidin-4-yl)isophthalate;
bis(1-oxyl-2,2,6,6-tetramethylpiperidin-4-yl)terephthalate;
bis(1-oxyl-2,2,6,6-tetramethylpiperidin-4-yl)hexahydroterephthalate;
N,N'-bis(1-oxyl-2,2,6,6-tetramethylpiperidin-4-yl)adipamide;
N-(1-oxyl-2,2,6,6-tetramethylpiperidin-4-yl)-caprolactam;
N-(1-oxyl-2,2,6,6-tetramethylpiperidin-4-yl)-dodecylsuccinimide;
2,4,6-tris-[N-butyl-N-(1-oxyl-2,2,6,6-tetramethylpiperidin-4-yl)]-s-triazine;
4,4'-ethylenebis(1-oxyl-2,2,6,6-tetramethylpiperazin-3-one); and the like.

As used herein, the abbreviation TEMPO stands for 2,2,6,6-tetramethyl-1-piperidinyloxy. Thus, 4-amino-TEMPO is 4-amino-2,2,6,6-tetramethyl-1-piperidinyloxy; 4-hydroxy-TEMPO is 4-hydroxy-2,2,6,6-tetramethyl-1-piperidinyloxy (also known in the art as HTEMPO); 4-oxo-TEMPO is 4-oxo-2,2,6,6-tetramethyl-1-piperidinyloxy; and so on.

It is preferred that at least one nitroxide free radical compound employed in the practice of the present invention be 4-amino-TEMPO, 4-oxo-TEMPO, 4-hydroxy-TEMPO, or TEMPO.

Blends of two or more of the foregoing, e.g., 4-amino-TEMPO and 4-oxo-TEMPO, can also be employed.

Such stable nitroxide free radical compounds can be prepared by known methods. (See, for example, U.S. Patent Numbers 3,163,677; 3,334,103; 3,372,182; 3,422,144; 3,494,930; 3,502,692; 3,873,564; 3,966,711; and 4,665,185; which are incorporated herein by reference.) They are suitable for use over a wide range of temperatures, but distillation temperatures employed with the ethylenically unsaturated monomers that are stabilized with the nitroxide free radical compounds typically range from about 60° C to about 180° C, preferably from about 70° C to about 165° C, and, more preferably, from about 80° C to about 150° C. Such distillations are generally performed at an absolute pressure in the range of about 10 to about 1,200 mm ofHg.

The ethylenically unsaturated monomer, the premature polymerization and polymer growth of which is an object of the present invention, can be any such monomer for which unintended polymerization and/or polymer growth during its manufacture, storage, and/or distribution is a problem. Among those monomers that will benefit from the practice of the present invention are: styrene, α-methylstyrene, styrene sulfonic acid, vinyltoluene, divinylbenzenes, polyvinylbenzenes, alkylated styrene, 2-vinylpyridine, acrylonitrile, methacrylonitrile, methyl acrylate, ethyl acrylate, methyl methacrylate, ethyl methacrylate, acrylic acid, methacrylic acid, butadiene, chloroprene, isoprene, and the like.

The ethylenically unsaturated monomers will not necessarily be stabilized indefinitely by the presence of the inhibitor(s), especially when the monomers are heated as in distillation, but they can be considered to be stabilized as long as A) there is a measurable increase in the time for which they can be heated before the onset of polymerization and/or polymer growth in a static system, B) the amount of polymer made at a constant temperature remains constant over time in a dynamic system, C) the rate of polymer growth is significantly slower than when the growth inhibiting system is not present, and/or D) the rate of polymer growth is nearly additive in the multi-pass test.

Those skilled in the art will understand that, if desired, free radical scavengers can also be included in the stabilization process. For example, air or O₂, as disclosed in U.S. Patent Numbers 5,545,782 and 5,545,786, can be added, as can the aromatic nitro compounds disclosed in U. S. Patent Number 5,254,760, the dihetero-substituted benzene compounds having at least one transferable hydrogen, e.g., a quinone derivative such as the mono-methyl-ether of hydroquinone disclosed in European Patent Application 0 765 856 A1, the iron compounds disclosed in WO 98/25872, and other inhibitors, e.g., phenolics and certain inorganic salts, well-known to those skilled in the art.

The polymerization inhibitor(s) can be introduced into the monomer to be protected by any conventional method. They can, for example, be added as a concentrated solution in suitable solvents just upstream from the point of desired application by any suitable means. In addition, individual inhibiting components can be injected separately into the distillation train along with the incoming feed and/or through separate and multiple entry points, provided there is an efficient distribution of the inhibiting composition. Since the inhibitors are gradually depleted during the distillation operation, it is generally advantageous to maintain the appropriate amount of them in the distillation apparatus by adding them during the course of the distillation process. Adding inhibitors can be done either on a generally continuous basis or intermittently, in order to maintain the inhibitor concentration above the minimum required level.

The total inhibitor concentration should be from about 1 to about 2000 ppm versus the monomer being inhibited; preferably from about 5 to about 1000 ppm, depending on the conditions of use.

The advantages and the important features of the present invention will be more apparent from the following examples.

### EXAMPLES

### Procedure for Multi-Pass Reboiler Test

### Preparation of First Pass Feed Solution:

*Tert*-butylcatechol (TBC) is removed from commercially available styrene by distillation under vacuum. Removal of TBC is verified by caustic titration. The desired amount of inhibitor(s) is added to this TBC-free styrene either directly or by first making a concentrated solution of the inhibitor in TBC-free styrene followed by further dilution with TBC-free styrene.

### Procedure for Reboiler Test (a dynamic test):

A quantity of the Feed Solution containing inhibitor (blend) at the desired charge (stated as a wt/wt total inhibitor to styrene) is added to a round-bottom flask (the Pot) and heated to the desired temperature (usually 130° C) and brought to reflux by adjusting the pressure/vacuum. Once the Pot contents are at temperature, a continuous stream of fresh Feed Solution is begun at a rate that will add the volume of the initial Pot solution to the Pot over a period oftime called the residence time (typically, one hour). At the same time that the fresh Feed Solution flow is begun, the Bottoms Stream flow is also begun. The Bottoms Stream is solution in the Pot that is removed at the same rate as the fresh Feed Solution is added. The equal flows of Feed and Bottoms Streams cause the quantity in the Pot to remain constant over the time of the experiment, while allowing continuous replenishment of inhibitor. This procedure simulates the way inhibitors are used in a distillation train of a plant producing vinyl monomers. The experiment continues with flow in and out of the Pot for a specified period of time. Typically, the First Pass runs for 10 hours, the Second Pass runs for 9 hours, the Third Pass runs for 8 hours, etc.

Samples are collected hourly from the Bottoms Stream. These samples are analyzed for polymer content via the methanol turbidity method. Polymer Content is reported as average weight percent polymer (Ave. Wt % Polymer), which is the average of the polymer contents for the hourly samples after four hours running. The amount of polymer in the samples is an indication of effectiveness of the inhibitor system being tested.

### Preparation of Second and Third Pass Feed Solutions:

The Bottoms Stream from the previous Pass is collected. The amounts of inhibitor(s) in the First Pass Feed Solution and the Bottoms Stream from the First Pass are determined by appropriate analytical method(s), e.g., gas chromatography. An amount of inhibitor(s) is added to the collective Bottoms Stream from the First Pass to increase the level of inhibitor(s) in the Bottoms Stream to a level equal to that found in the First Pass Feed Solution. For each subsequent Pass, an appropriate amount of inhibitor(s) is added to the collective Bottoms Streams to bring inhibitor(s) level equal to that found in the First Pass Feed Solution.

### Evaluation of the Results

The difference in the amount of polymer made in one Pass versus subsequent Passes is an indication of the ability of the inhibiting system to prevent or allow polymer to grow. For example, an increase in the amount of polymer made going from one Pass to the next which is roughly equivalent to the amount of polymer made during the First Pass is an indication that the inhibiting system effectively prevents polymer growth during recycle. Conversely, an increase in the amount of polymer made going from one Pass to the next that is dramatically greater (about 10 times or more) than the amount of polymer made during the First Pass is an indication that the inhibiting system does not effectively prevent polymer growth during recycle.

### Recycle of Nitroxyl-containing Streams at Low Temperatures

The Multi-Pass test described above simulates recycle of styrene purification tar streams in a plant. As stated above, many companies recycle certain process streams containing the polymerization inhibitor in order to improve their economics by reusing the inhibitor left in the process stream. It has previously been noted that the recycle of streams utilizing nitroxyls as polymerization inhibitors in higher temperature (above 115° C) plants causes loss of inhibitor efficiency, such that recycle leads to higher polymer content than expected or desired. It has now been found that the recycle of streams utilizing nitroxyls as polymerization inhibitors in lower temperature plants (below about 110° C) can be done effectively without loss of inhibitor efficiency, as indicated by comparison of test results versus DNBP or a PDA/DNBP blend, both of which have been shown to be effectively utilized with tar recycle in low temperature and high temperature plants. The test results in Table 1 below illustrate the invention.

| Table 1 | | | | |
|---|---|---|---|---|
| Inhibitor System | Temp (°C) | Ave. Wt % Polymer | | |
| | | Pass 1 | Pass 2 | Pass 3 |
| 1500 ppm PDA/DNBP Blend | 130 | 0.066 | 0.183 | 0.423 |
| 1500 ppm DNBP | 130 | 0.109 | 0.266 | 0.430 |
| 300 ppm Nitroxyl | 130 | 0.052 | 1.45 | 7.45 |
| 100 ppm Nitroxyl | 116 | 0.00051 | 0.0408 | 2.36 |
| 25 ppm Nitroxyl | 100 | 0.00019 | 0.00022 | 0.00043 |

| | | | | |
|---|---|---|---|---|
| PDA is N-(1,4-dimethylpentyl)-N'-phenyl-*p*-phenylenediamine. DNBP is 2,4-dinitro-6-*sec*-butylphenol. Nitroxyl is 4-oxo-TEMPO. | | | | |

The first two Examples indicate results for systems known in industry to recycle with excellent inhibitor efficiency. The third example reflects conditions known in industry to lead to loss of nitroxyl inhibitor efficiency. It will be noted in the above Table that as the temperature is lowered, not only does nitroxyl inhibitor efficiency improve in recycle, but also the amount of nitroxyl utilized can be lowered providing an additional advantage owing to the relatively high cost of the nitroxyl compounds.

In view of the many changes and modifications that can be made without departing from principles underlying the invention, reference should be made to the appended claims for an understanding of the scope of the protection to be afforded the invention.

## Claims

1. A process for the production and purification of unsaturated monomers, said process employing nitroxyl-containing inhibitors wherein process streams containing the inhibitor are recycled, whereby said streams are recycled at a reboiler temperature of below 110° C.

2. The process of claim 1 wherein the nitroxyl-containing inhibitor is of the following structural formula: 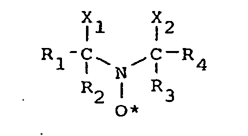 wherein
R₁ and R₄ are independently selected from the group consisting of hydrogen, alkyl, and heteroatom-substituted alkyl;
R₂ and R₃ are independently selected from the group consisting of alkyl and heteroatom-substituted alkyl; and
X₁ and X₂
(1) are independently selected from the group consisting of halogen, cyano, amido, -S-C₆H₅, carbonyl, alkenyl, alkyl of 1 to 15 carbon atoms, COOR₇, -S-COR₇, and -OCOR₇, wherein R₇ is alkyl or aryl, or
(2) taken together, form a ring structure with the nitrogen.

3. The process of claim 1 wherein said monomers contain impurities from the monomer production and/or purification processes.

4. The process of claim 3 wherein the impurities include polymer formed during the production and/or purification processes.

5. The process of claim 4 wherein the polymer formed during the production and/or purification processes is soluble in the monomer stream.

6. The process of claim 4 wherein the polymer formed during the production and/or purification processes is insoluble in the monomer stream.

7. The process of claim 1 wherein said monomers are undergoing purification by distillation.

8. The process of claim 7 wherein the distillation process occurs at pressures less than 760 mm Hg.

9. The process of claim 7 wherein the distillation process is a continuous process.

10. The process of claim 4 wherein the equipment in which the distillation process occurs contains polymer.

11. The process of claim 10 wherein the polymer was formed during the monomer's production and/or purification processes.

12. The process of claim 10 wherein the polymer is not dissolved in the monomer stream.

13. The process of claim 7 wherein said monomers contain impurities from the monomer production and/or purification processes.

14. The process of claim 13 wherein the impurities include polymer formed during the production and/or purification processes.

15. The process of claim 14 wherein the polymer formed during the production and/or purification processes is soluble in the monomer stream.

16. The process of claim 14 wherein the polymer formed during the production and/or purification processes is insoluble in the monomer stream.

17. The process of claim 2 wherein the nitroxyl-containing inhibitor is of the structure 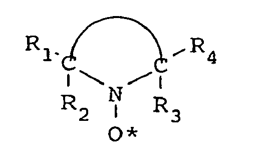 wherein R₁ and R₄ are independently selected from the group consisting of hydrogen, alkyl, and heteroatom-substituted alkyl and R₂ and R₃ are independently selected from the group consisting of alkyl and heteroatom-substituted alkyl, and the 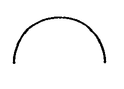 portion represents the atoms necessary to form a five-, six-, or seven-membered heterocyclic ring.

18. The process of claim 2 wherein the inhibitor is a blend of two nitroxyls.

19. The process of claim 17 wherein the inhibitor contains one or more nitroxyls selected from the group consisting of:
N,N-di-*tert*-butylnitroxide;
N,N-di-*tert*-amylnitroxide;
N-*tert*-butyl-2-methyl-1-phenyl-propylnitroxide;
N-*tert*-butyl-1-diethylphosphono-2,2-dimethylpropylnitroxide;
2,2,6,6-tetramethyl-piperidinyloxy;
4-amino-2,2,6,6-tetramethyl-piperidinyloxy;
4-hydroxy-2,2,6,6-tetramethyl-piperidinyloxy;
4-oxo-2,2,6,6-tetramethyl-piperidinyloxy;
4-dimethylamino-2,2,6,6-tetramethyl-piperidinyloxy;
4-ethanoyloxy-2,2,6,6-tetramethyl-piperldinyloxy;
2,2,5,5-tetramethylpyrrolidinyloxy;
3-amino-2,2,5,5-tetramethylpyrrolidinyloxy;
2,2,4,4-tetramethyl-1-oxa-3-azacyclopentyl-3-oxy;
2,2,4,4-tetramethyl-1-oxa-3-pyrrolinyl-1-oxy-3-carboxylic acid;
2,2,3,3,5,5,6,6-octamethyl-1,4-diazacyclohexyl-1,4-dioxy;
4-bromo-2,2,6,6-tetramethyl-piperidinyloxy;
4-chloro-2,2,6,6-tetramethyl-piperidinyloxy;
4-iodo-2,2,6,6-tetramethyl-piperidinyloxy;
4-fluoro-2,2,6,6-tetramethyl-piperidinyloxy;
4-cyano-2,2,6,6-tetramethyl-piperidinyloxy;
4-carboxy-2,2,6,6-tetramethyl-piperidinyloxy;
4-carbomethoxy-2,2,6,6-tetramethyl-piperidinyloxy;
4-carbethoxy-2,2,6,6-tetramethyl-piperidinyloxy;
4-cyano-4-hydroxy-2,2,6,6-tetramethyl-piperidinyloxy;
4-methyl-2,2,6,6-tetramethyl-piperidinyloxy;
4-carbethoxy-4-hydroxy-2,2,6,6-tetramethyl-piperidinyloxy;
4-hydroxy-4-(1-hydroxypropyl)-2,2,6,6-tetramethyl-piperldinyloxy;
4-methyl-2,2,6,6-tetramethyl-1,2,5,6-tetrahydropyridine-1-oxyl;
4-carboxy-2,2,6,6-tetramethyl-1,2,5,6-tetrahydropyridine -1-oxyl;
4-carbomethoxy-2,2,6,6-tetramethyl-1,2,5,6-tetrahydropyridine -1-oxyl;
4-carbethoxy-2,2,6,6-tetramethyl-1,2,5,6-tetrahydropyridine -1-oxyl;
4-amino-2,2,6,6-tetramethyl-1,2,5,6-tetrahydropyridine -1-oxyl;
4-amido-2,2,6,6-tetramethyl-1,2,5,6-tetrahydropyridine -1-oxyl;
3,4-diketo-2,2,5,5-tetramethylpyrrolidinyloxy;
3-keto-4-oximino-2,2,5,5-tetramethylpyrrolidinyloxy;
3-keto-4-benzylidine-2,2,5,5-tetramethylpyrrolidinyloxy;
3-keto-4,4-dibromo-2,2,5,5-tetramethylpyrrolidinyloxy;
2,2,3,3,5,5-hexamethylpyrrolidinyloxy;
3-carboximido-2,2,5,5-tetramethylpyrrolidinyloxy;
3-oximino-2,2,5,5-tetramethylpyrrolidinyloxy;
3-hydroxy-2,2,5,5-tetramethylpyrrolidinyloxy;
3-cyano-3-hydroxy-2,2,5,5-tetramethylpyrrolidinyloxy;
3-carbomethoxy-3-hydroxy-2,2,5,5-tetramethylpyrrolidinyloxy;
3-carbethoxy-3-hydroxy-2,2,5,5-tetramethylpyrrolidinyloxy;
2,2,5,5-tetramethyl-3-carboxamido-2,5-dihydropyrrole-1-oxyl;
2,2,5,5-tetramethyl-3-amino-2,5-dihydropyrrole-1-oxyl;
2,2,5,5-tetramethyl-3-carbethoxy-2,5-dihydropyrrole-1-oxyl;
2,2,5,5-tetramethyl-3-cyano-2,5-dihydropyrrole-1-oxyl;
bis(1-oxyl-2,2,6,6-tetramethylpiperidin-4-yl)succinate;
bis(1-oxyl-2,2,6,6-tetramethylpiperidin-4-yl)adipate;
bis(1-oxyl-2,2,6,6-tetramethylpiperidin-4-yl)sebacate;
bis(1-oxyl-2,2,6,6-tetramethylpiperidin-4-yl)n-butylmalonate;
bis(1-oxyl-2,2,6,6-tetramethylpiperidin-4-yl)phthalate;
bis(1-oxyl-2,2,6,6-tetramethylpiperidin-4-yl)isophthalate;
bis(1-oxyl-2,2,6,6-tetramethylpiperidin-4-yl)terephthalate;
bis(1-oxyl-2,2,6,6-tetramethylpiperidin-4-yl)hexahydroterephthalate;
N,N'-bis(I -oxyl-2,2,6,6-tetramethylpiperidin-4-yl)adipamide;
N-(1-oxyl-2,2,6,6-tetramethylpiperidin-4-yl)-caprolactam;
N-(1-oxyl-2,2,6,6-tetramethylpiperidin-4-yl)-dodecylsuccinimide;
2,4,6-tris-[N-butyl-N-(1-oxyl-2,2,6,6-tetramethylpiperidin-4-yl)]-s-triazine; and
4,4'-ethylenebis(1-oxyl-2,2,6,6-tetramethylpiperazin-3-one).

## Patentansprüche

1. Verfahren zur Herstellung und Reinigung von ungesättigten Monomeren, wobei das Verfahren Nitroxyl-enthaltende Inhibitoren einsetzt, wobei Verfahrensströme, die den Inhibitor enthalten, recycelt werden, wobei die Ströme bei einer Verdampfertemperatur von unter 110 °C recycelt werden.

2. Verfahren nach Anspruch 1, wobei der Nitroxyl-enthaltende Inhibitor die folgende Strukturformel aufweist: 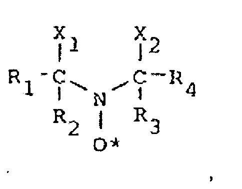 wobei
R₁ und R₄ unabhängig ausgewählt sind aus der Gruppe, bestehend aus Wasserstoff, Alkyl und Heteroatom-substituiertem Alkyl;
R₂ und R₃ unabhängig ausgewählt sind aus der Gruppe, bestehend aus Alkyl und Heteroatom-substituiertem Alkyl; und
X₁ und X₂
(1) unabhängig ausgewählt sind aus der Gruppe, bestehend aus Halogen, Cyano, Amido, -S-C₆H₅, Carbonyl, Alkenyl, Alkyl mit 1 bis 15 Kohlenstoffatomen, COOR₇, -S-COR₇ und -OCOR₇, wobei R₇ für Alkyl oder Aryl steht, oder
(2) zusammengenommen eine Ringstruktur mit dem Stickstoff bilden.

3. Verfahren nach Anspruch 1, wobei die Monomeren Verunreinigungen aus den Monomerherstellungs- und/oder -reinigungsverfahren enthalten.

4. Verfahren nach Anspruch 3, wobei die Verunreinigungen Polymer einschließen, das gebildet wird während der Herstellungs- und/oder Reinigungsverfahren.

5. Verfahren nach Anspruch 4, wobei das während der Herstellungs- und/oder Reinigungsverfahren gebildete Polymer in dem Monomerstrom löslich ist.

6. Verfahren nach Anspruch 4, wobei das während der Herstellungs- und/oder Reinigungsverfahren gebildete Polymer in dem Monomerstrom unlöslich ist.

7. Verfahren nach Anspruch 1, wobei die Monomere einer Reinigung durch Destillation unterworfen werden.

8. Verfahren nach Anspruch 7, wobei das Destillationsverfahren bei Drücken von weniger als 760 mm Hg stattfindet.

9. Verfahren nach Anspruch 7, wobei das Destillationsverfahren ein kontinuierliches Verfahren ist.

10. Verfahren nach Anspruch 4, wobei die Anlage, in der das Destillationsverfahren stattfindet, Polymer enthält.

11. Verfahren nach Anspruch 10, wobei das Polymer während der Herstellungs- und/oder Reinigungsverfahren des Monomers gebildet wurde.

12. Verfahren nach Anspruch 10, wobei das Polymer nicht in dem Monomerstrom gelöst ist.

13. Verfahren nach Anspruch 7, wobei die Monomere Verunreinigungen aus den Monomerherstellungs- und/oder -reinigungsverfahren enthalten.

14. Verfahren nach Anspruch 13, wobei die Verunreinigungen Polymer einschließen, das während der Herstellungs- und/oder Reinigungsverfahren gebildet wird.

15. Verfahren nach Anspruch 14, wobei das während der Herstellungs- und/oder Reinigungsverfahren gebildete Polymer in dem Monomerstrom löslich ist.

16. Verfahren nach Anspruch 14, wobei das während der Herstellungs- und/oder Reinigungsverfahren gebildete Polymer in dem Monomerstrom unlöslich ist.

17. Verfahren nach Anspruch 2, wobei der Nitroxyl-enthaltende Inhibitor die folgende Struktur aufweist: 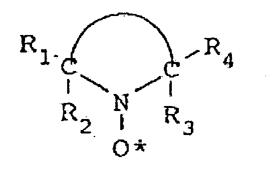 wobei R₁ und R₄ unabhängig ausgewählt sind aus der Gruppe, bestehend aus Wasserstoff, Alkyl und Heteroatom-substituiertem Alkyl und R₂ und R₃ unabhängig ausgewählt sind aus der Gruppe, bestehend aus Alkyl und Heteroatom-substituiertem Alkyl und der folgende Teil 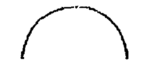 für die Atome steht, die notwendig sind, um einen fünf-, sechs- oder siebengliedrigen heterocyclischen Ring zu bilden.

18. Verfahren nach Anspruch 2, wobei der Inhibitor für ein Gemisch aus zwei Nitroxylen steht.

19. Verfahren nach Anspruch 17, wobei der Inhibitor ein oder mehrere Nitroxyl(e) enthält, das/die ausgewählt ist/sind aus der Gruppe, bestehend aus:
N, N-Di-tert.-butylnitroxid;
N, N-Di-tert.-amylnitroxid;
N-tert.-Butyl-2-methyl-1-phenyl-propylnitroxid;
N-tert.-Butyl-1-diethylphosphono-2,2-dimethylpropylnitroxid;
2,2,6,6-Tetramethyl-piperidinyloxy;
4-Amino-2,2,6,6-tetramethyl-piperidinyloxy;
4-Hydroxy-2,2,6,6-tetramethyl-piperidinyloxy;
4-Oxo-2,2,6,6-tetramethyl-piperidinyloxy;
4-Dimethylamino-2,2,6,6-tetramethyl-piperidinyloxy;
4-Ethanoyloxy-2,2,6,6-tetramethyl-piperidinyloxy;
2,2,5,5-Tetramethylpyrrolidinyloxy;
3-Amino-2,2,5,5-tetramethylpyrrolidinyloxy;
2,2,4,4-Tetramethyl-1-oxa-3-azacyclopentyl-3-oxy;
2,2,4,4-Tetramethyl-1-oxa-3-pyrrolinyl-1-oxy-3-carbonsäure;
2,2,3,3,5,5,6,6-Octamethyl-1,4-diazacyclohexyl-1,4-dioxy;
4-Brom-2,2,6,6-tetramethyl-piperidinyloxy;
4-Chlor-2,2,6,6-tetramethyl-piperidinyloxy;
4-lod-2,2,6,6-tetramethyl-piperidinyloxy;
4-Fluor-2,2,6,6-tetramethyl-piperidinyloxy;
4-Cyano-2,2,6,6-tetramethyl-piperidinyloxy;
4-Carboxy-2,2,6,6-tetramethyl-piperidinyloxy;
4-Carbomethoxy-2,2,6,6-tetramethyl-piperidinyloxy;
4-Carbethoxy-2,2,6,6-tetramethyl-piperidinyloxy;
4-Cyano-4-hydroxy-2,2,6,6-tetramethyl-piperidinyloxy;
4-Methyl-2,2,6,6-tetramethyl-piperidinyloxy;
4-Carbethoxy-4-hydroxy-2,2,6,6-tetramethyl-piperidinyloxy;
4-Hydroxy-4-(1-hydroxypropyl)-2,2,6,6-tetramethyl-piperidinyloxy;
4-Methyl-2,2,6,6-tetramethyl-1,2,5,6-tetrahydropyridin-1-oxyl;
4-Carboxy-2,2,6,6-tetramethyl-1,2,5,6-tetrahydropyridin-1-oxyl;
4-Carbomethoxy-2,2,6,6-tetramethyl-1,2,5,6-tetrahydropyridin-1-oxyl;
4-Carbethoxy-2,2,6,6-tetramethyl-1,2,5,6-tetrahydropyridin-1-oxyl;
4-Amino-2,2,6,6-tetramethyl-1,2,5,6-tetrahydropyridin-1-oxyl;
4-Amido-2,2,6,6-tetramethyl-1,2,5,6-tetrahydropyridin-1-oxyl;
3,4-Diketo-2,2,5,5-tetramethylpyrrolidinyloxy;
3-Keto-4-oximino-2,2,5,5-tetramethylpyrrolidinyloxy;
3-Keto-4-benzylidin-2,2,5,5-tetramethylpyrrolidinyloxy;
3-Keto-4,4-dibrom-2,2,5,5-tetramethylpyrrolidinyloxy;
2,2,3,3,5,5-Hexamethylpyrrolidinyloxy;
3-Carboximido-2,2,5,5-tetramethylpyrrolidinyloxy;
3-Oximino-2,2,5,5-tetramethylpyrrolidinyloxy;
3-Hydroxy-2,2,5,5-tetramethylpyrrolidinyloxy;
3-Cyano-3-hydroxy-2,2,5,5-tetramethylpyrrolidinyloxy;
3-Carbomethoxy-3-hydroxy-2,2,5,5-tetramethylpyrrolidinyloxy;
3-Carbethoxy-3-hydroxy-2,2,5,5-tetramethylpyrrolidinyloxy;
2,2,5,5-Tetramethyl-3-carboxamido-2,5-dihydropyrrol-1-oxyl;
2,2,5,5-Tetramethyl-3-amino-2,5-dihydropyrrol-1-oxyl;
2,2,5,5-Tetramethyl-3-carbethoxy-2,5-dihydropyrrol-1-oxyl;
2,2,5,5-Tetramethyl-3-cyano-2,5-dihydropyrrol-1-oxyl;
Bis(1-oxyl-2,2,6,6-tetramethylpiperidin-4-yl)succinat;
Bis(1-oxyl-2,2,6,6-tetramethylpiperidin-4-yl)adipat;
Bis(1-oxyl-2,2,6,6-tetramethylpiperidin-4-yl)sebacat;
Bis(1-oxyl-2,2,6,6-tetramethylpiperidin-4-yl)n-butylmalonat;
Bis(1-oxyl-2,2,6,6-tetramethylpiperidin-4-yl)phthalat;
Bis(1-oxyl-2,2,6,6-tetramethylpiperidin-4-yl)isophthalat;
Bis(1-oxyl-2,2,6,6-tetramethylpiperidin-4-yl)terephthalat;
Bis(1-oxyl-2,2,6,6-tetramethylpiperidin-4-yl)hexahydroterephthalat;
N,N'-Bis(1-oxyl-2,2,6,6-tetramethylpiperidin-4-yl)adipamid;
N-(1-Oxyl-2,2,6,6-tetramethylpiperidin-4-yl)-caprolactam;
N-(1-Oxyl-2,2,6,6-tetramethylpiperidin-4-yl)-dodecylsuccinimid;
2,4,6-Tris-[N-butyl-N-(1-oxyl-2,2,6,6-tetramethylpiperidin-4-yl)]-s-triazin; und
4,4'-Ethylenbis(1-oxyl-2,2,6,6-tetramethylpiperazin-3-on).

## Revendications

1. Procédé de production et de purification de monomères insaturés, ledit procédé employant des inhibiteurs contenant un nitroxyle, dans lequel des courants de procédé contenant l'inhibiteur sont recyclés, moyennant quoi lesdits courants sont recyclés à une température de rebouilleur en dessous de 110°C.

2. Procédé selon la revendication 1, dans lequel l'inhibiteur contenant un nitroxyle est de la formule structurelle suivante : 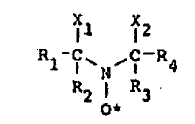 dans laquelle
R₁ et R₄ sont indépendamment choisis dans le groupe constitué par un atome d'hydrogène, un groupe alkyle et alkyle substitué par hétéroatome ;
R₂ et R₃ sont indépendamment choisis dans le groupe constitué par le groupe alkyle et alkyle substitué par hétéroatome ; et
X₁ et X₂
(1) sont indépendamment choisis dans le groupe constitué par un atome d'halogène, un groupe cyano, amido, -S-C₆H₅, carbonyle, alcényle, alkyle de 1 à 15 atomes de carbone, COOR₇, -S-COR₇ et -OCOR₇, dans lesquels R₇ est un groupe alkyle ou aryle, ou
(2) pris ensemble, forment une structure de cycle avec l'atome d'azote.

3. Procédé selon la revendication 1, dans lequel lesdits monomères contiennent des impuretés provenant des procédés de production et/ou de purification de monomères.

4. Procédé selon la revendication 3, dans lequel les impuretés comprennent un polymère formé pendant les procédés de production et/ou de purification.

5. Procédé selon la revendication 4, dans lequel le polymère formé pendant les procédés de production et/ou de purification est soluble dans le courant de monomères.

6. Procédé selon la revendication 4, dans lequel le polymère formé pendant les procédés de production et/ou de purification est insoluble dans le courant de monomères.

7. Procédé selon la revendication 1, dans lequel lesdits monomères subissent une purification par distillation.

8. Procédé selon la revendication 7, dans lequel le procédé de distillation se produit à des pressions inférieures à 760 mm Hg.

9. Procédé selon la revendication 7, dans lequel le procédé de distillation est un procédé continu.

10. Procédé selon la revendication 4, dans lequel l'équipement dans lequel le procédé de distillation se produit contient un polymère.

11. Procédé selon la revendication 10, dans lequel le polymère était formé pendant les procédés de production et/ou de purification de monomères.

12. Procédé selon la revendication 10, dans lequel le polymère n'est pas dissous dans le courant de monomères.

13. Procédé selon la revendication 7, dans lequel lesdits monomères contiennent des impuretés provenant des procédés de production et/ou de purification de monomères.

14. Procédé selon la revendication 13, dans lequel les impuretés comprennent un polymère formé pendant les procédés de production et/ou de purification.

15. Procédé selon la revendication 14, dans lequel le polymère formé pendant les procédés de production et/ou de purification est soluble dans le courant de monomères.

16. Procédé selon la revendication 14, dans lequel le polymère formé pendant les procédés de production et/ou de purification est insoluble dans le courant de monomères.

17. Procédé selon la revendication 2, dans lequel l'inhibiteur contenant un nitroxyle est de structure 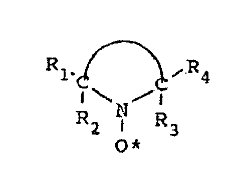 dans laquelle R₁ et R₄ sont indépendamment choisis dans le groupe constitué par un atome d'hydrogène, un groupe alkyle et alkyle substitué par hétéroatome, et R₂ et R₃ sont indépendamment choisis dans le groupe constitué par un groupe alkyle et alkyle substitué par hétéroatome, et la portion 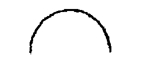 représente les atomes nécessaires pour former un cycle hétérocyclique à 5, 6 ou 7 chaînons.

18. Procédé selon la revendication 2, dans lequel l'inhibiteur est un mélange de deux groupes nitroxyle.

19. Procédé selon la revendication 17, dans lequel l'inhibiteur contient un ou plusieurs groupes nitroxyle choisis dans le groupe constitué par :
le N,N-di-tert-butylnitroxyde ;
le N,N-di-tert-amylnitroxyde ;
le N-tert-butyl-2-méthyl-1-phényl-propylnitroxyde ;
le N-tert-butyl-1-diéthylphosphono-2,2-diméthylpropylnitroxyde ;
le 2,2,6,6-tétraméthyl-pipéridinyloxy ;
le 4-amino-2,2,6,6-tétraméthyl-pipéridinyloxy ;
le 4-hydroxy-2,2,6,6-tétraméthyl-pipéridinyloxy ;
le 4-oxo-2,2,6,6-tétraméthyl-pipéridinyloxy ;
le 4-diméthylamino-2,2,6,6-tétraméthyl-pipéridinyloxy ;
le 4-éthanoyloxy-2,2,6,6-tétraméthyl-pipéridinyloxy ;
le 2,2,5,5-tétraméthylpyrrolidinyloxy ;
le 3-amino-2,2,5,5-tétraméthylpyrrolidinyloxy ;
le 2,2,4,4-tétraméthyl-1-oxa-3-azacyclopentyl-3-oxy ;
l'acide 2,2,4,4-tétraméthyl-1-oxa-3-pyrrolinyl-1-oxy-3-carboxylique ;
le 2,2,3,3,5,5,6,6-octaméthyl-1,4-diazacyclohexyl-1,4-dioxy ;
le 4-bromo-2,2,6,6-tétraméthyl-pipéridinyloxy ;
le 4-chloro-2,2,6,6-tétraméthyl-pipéridinyloxy ;
le 4-iodo-2,2,6,6-tétraméthyl-pipéridinyloxy ;
le 4-fluoro-2,2,6,6-tétraméthyl-pipéridinyloxy ;
le 4-cyano-2,2,6,6-tétraméthyl-pipéridinyloxy ;
le 4-carboxy-2,2,6,6-tétraméthyl-pipéridinyloxy ;
le 4-carbométhoxy-2,2,6,6-tétraméthyl-pipéridinyloxy ;
le 4-carbéthoxy-2,2,6,6-tétraméthyl-pipéridinyloxy ;
le 4-cyano-4-hydroxy-2,2,6,6-tétraméthyl-pipéridinyloxy ;
le 4-méthyl-2,2,6,6-tétraméthyl-pipéridinyloxy ;
le 4-carbéthoxy-4-hydroxy-2,2,6,6-tétraméthyl-pipéridinyloxy ;
le 4-hydroxy-4-(1-hydroxypropyl)-2,2,6,6-tétraméthyl-pipéridinyloxy ;
le 4-méthyl-2,2,6,6-tétraméthyl-1,2,5,6-tétrahydropyridine-1-oxyle ;
le 4-carboxy-2,2,6,6-tétraméthyl-1,2,5,6-tétrahydropyridine-1-oxyle ;
le 4-carbométhoxy-2,2,6,6-tétraméthyl-1,2,5,6-tétrahydropyridine-1-oxyle ;
le 4-carbéthoxy-2,2,6,6-tétraméthyl-1,2,5,6-tétrahydropyridine-1-oxyle ;
le 4-amino-2,2,6,6-tétraméthyl-1,2,5,6-tétrahydropyridine-1-oxyle ;
le 4-amido-2,2,6,6-tétraméthyl-1,2,5,6-tétrahydropyridine-1-oxyle ;
le 3,4-dicéto-2,2,5,5-tétraméthylpyrrolidinyloxy ;
le 3-céto-4-oximino-2,2,5,5-tétraméthylpyrrolidinyloxy ;
le 3-céto-4-benzylidine-2,2,5,5-tétraméthylpyrrolidinyloxy ;
le 3-céto-4,4-dibromo-2,2,5,5-tétraméthylpyrrolidinyloxy ;
le 2,2,3,3,5,5-hexaméthylpyrrolidinyloxy ;
le 3-carboximido-2,2,5,5-tétraméthylpyrrolidinyloxy ;
le 3-oximino-2,2,5,5-tétraméthylpyrrolidinyloxy ;
le 3-hydroxy-2,2,5,5-tétraméthylpyrrolidinyloxy ;
le 3-cyano-3-hydroxy-2,2,5,5-tétraméthylpyrrolidinyloxy ;
le 3-carbométhoxy-3-hydroxy-2,2,5,5-tétraméthylpyrrolidinyloxy ;
le 3-carbéthoxy-3-hydroxy-2,2,5,5-tétraméthylpyrrolidinyloxy ;
le 2,2,5,5-tétraméthyl-3-carboxamido-2,5-dihydropyrrole-1-oxyle ;
le 2,2,5,5-tétraméthyl-3-amino-2,5-dihydropyrrole-1-oxyle ;
le 2,2,5,5-tétraméthyl-3-carbéthoxy-2,5-dihydropyrrole-1-oxyle ;
le 2,2,5,5-tétraméthyl-3-cyano-2,5-dihydropyrrole-1-oxyle ;
le succinate de bis(1-oxyl-2,2,6,6-tétraméthylpipéridin-4-yle) ;
l'adipate de bis(1-oxyl-2,2,6,6-tétraméthylpipéridin-4-yle) ;
le sébacate de bis(1-oxyl-2,2,6,6-tétraméthylpipéridin-4-yle) ;
le n-butylmalonate de bis(1-oxyl-2,2,6,6-tétraméthylpipéridin-4-yle) ;
le phtalate de bis(1-oxyl-2,2,6,6-tétraméthylpipéridin-4-yle) ;
l'isophtalate de bis(1-oxyl-2,2,6,6-tétraméthylpipéridin-4-yle) ;
le téréphtalate de bis(1-oxyl-2,2,6,6-tétraméthylpipéridin-4-yle) ;
l'hexahydrotéréphtalate de bis(1-oxyl-2,2,6,6-tétraméthylpipéridin-4-yle) ;
le N,N'-bis(1-oxyl-2,2,6,6-tétraméthylpipéridin-4-yl)adipamide ;
le N-(1-oxyl-2,2,6,6-tétraméthylpipéridin-4-yl)-caprolactame ;
le N-(1-oxyl-2,2,6,6-tétraméthylpipéridin-4-yl)-dodécylsuccinimide ;
la 2,4,6-tris[N-butyl-N-(1-oxyl-2,2,6,6-tétraméthylpipéridin-4-yl)]-s-triazine ; et
la 4,4'éthylènebis(1-oxyl-2,2,6,6-tétraméthylpipérazin-3-one).
